(19) Europäisches Patentamt
European Patent Office
Office européen des brevets

(11) Veröffentlichungsnummer : **0 526 531 B1**

(12) # EUROPÄISCHE PATENTSCHRIFT

(45) Veröffentlichungstag der Patentschrift : **22.06.94 Patentblatt 94/25**

(51) Int. Cl.$^5$ : **A61K 9/127**

(21) Anmeldenummer : **91908541.5**

(22) Anmeldetag : **25.04.91**

(86) Internationale Anmeldenummer : **PCT/EP91/00796**

(87) Internationale Veröffentlichungsnummer : **WO 91/16880 14.11.91 Gazette 91/26**

(54) **LIPOSOMEN MIT POSITIVER ÜBERSCHUSSLADUNG.**

(30) Priorität : **27.04.90 DE 4013632**

(43) Veröffentlichungstag der Anmeldung : **10.02.93 Patentblatt 93/06**

(45) Bekanntmachung des Hinweises auf die Patenterteilung : **22.06.94 Patentblatt 94/25**

(84) Benannte Vertragsstaaten : **AT BE CH DE DK ES FR GB GR IT LI LU NL SE**

(56) Entgegenhaltungen :
**EP-A- 018 772**
**EP-A- 0 002 202**
**EP-A- 0 088 046**
**WO-A-90/00555**
**Chemical Abstracts, vol.23, 1929 (Columbus, Ohio, US) Weitzmann et al.:"Prepatation of glycerides of amino fatty acids"**
**Patent Abstracts of Japan, vol. 12, No 202 (C-503)(3049), 10 June 1988**

(73) Patentinhaber : **Max-Planck-Gesellschaft zur Förderung der Wissenschaften e.V.**
**Bunsenstrasse 10**
**D-37073 Göttingen (DE)**

(72) Erfinder : **EIBL, Hansjörg**
**Heinrich-Deppe-Ring 22**
**D-3406 Bovenden (DE)**
Erfinder : **KAUFMANN-KOLLE, Petra**
**Gehrenweg 13**
**D-3407 Gleichen-Diemarden (DE)**
Erfinder : **UNGER, Clemens**
**Herzberger Landstrasse 2b**
**D-3400 Göttingen (DE)**

(74) Vertreter : **Huber, Bernhard, Dipl.-Chem.**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

EP 0 526 531 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Liposomen mit positiver Überschußladung, die als Wirkstoffträger bei der Behandlung von Lebererkrankungen eingesetzt werden können.

Liposomen sind kugelförmige Gebilde aus einer oder mehreren Lipiddoppelschichten mit wäßrigem Innenraum (Lipidvesikeln). Derartige Bläschen lassen sich durch mechanische Feinstverteilung von Phospholipiden (z.B. Lecithin) in wäßrigen Medien herstellen.

Von Bangham et al., J.Mol.Biol. 13 (1965) 238-252, wurde beobachtet, daß Phospholipide in Gegenwart von Wasser Überstrukturen ausbilden. In Abhängigkeit von physikalischen Parametern wie Druck, Temperatur und Ionenkonzentration formen sich Mizellen, unilamellare bzw. multilamellare Liposomen oder auch einfache Lipiddoppelschichten (vgl. Liposomes: From physical structure to therapeutic application (1981), Knight, C.G. (Ed.), Elsevier, North Holland Biomedical Press, Kapitel 2: H. Eibl, Phospholipid synthesis, 19-50; Kapitel 3: F. Szoka und D. Papahadjopoulos, Liposomes: Preparation and characterization, 51-104). Kleine, unilamellare Liposomen sind kugelförmige Gebilde mit einem Durchmesser von 20 bis 200 nm (vgl. Barenholtz et al., FEBS Lett. 99 (1979) 210-214). Ihr Innenvolumen besteht aus Wasser, das durch die Lipiddoppelschicht nach außen abgegrenzt ist. Je nach Lipophilie oder Hydrophilie können Wirkstoffe entweder in der Lipiddoppelschicht oder im wäßrigen Innenvolumen der Liposomen eingeschlossen und im Organismus über die Körperflüssigkeiten transportiert und verteilt werden.

Aufgrund ihrer Struktur dienen Liposomen in der Biochemie und Molekularbiologie als Membranmodelle. In den vergangenen Jahren sind außerdem zahlreiche Arbeiten über die Eigenschaften von Liposomen und ihre Verwendung als Arzneistoffträger veröffentlicht worden (vgl. z.B. H. Schreiner und M. Raeder-Schikorr, Pharmazie in unserer Zeit 11 (1982) 97-108). Bisher publizierte Tierversuche zeigen generell, daß Leber und Milz bezüglich der Aufnahme von Liposomen über andere Organe dominieren. Etwa 8 % der Liposomen werden nach einer Stunde und etwa 15 % nach 24 Stunden in der Leber vorgefunden. Die Unterschiede in der Organverteilung und in der Organanreicherung zwischen Liposomen, die neutral sind oder solchen, die eine 10 %ige negative Überschußladung tragen, sind allerdings gering.

Die mögliche Verwendung von Liposomen in der Medizin zielt im wesentlichen auf die selektive Behandlung von Krankheiten ab. Die gewünschten Wirkungen des in Liposomen eingeschlossenen Wirkstoffes sollen gefördert werden, die unerwünschten Wirkungen dagegen vermindert werden (Verbesserung des therapeutischen Index). Überzeugend ist ein solches Konzept bei der Behandlung einer isolierten Erkrankung, die auf ein bestimmtes Organ beschränkt ist, wobei Lebererkrankungen hierbei eine besondere Bedeutung zukommt. Sofern die eingeschlossenen Wirkstoffe ausschließlich in die Leber gelangen, nicht aber unspezifisch über den gesamten Organismus verteilt werden, wäre die Behandlung des erkrankten Organes eine weitgehend spezifische. Die nachfolgenden zwei Beispiele aus der inneren Medizin verdeutlichen diese Vorstellungen und weisen auf die praktische Bedeutung dieses Prinzips hin:

Beispiel 1: Maligne Lebererkrankungen

Bei der Metastasierung von bösartigen Tumoren kann die Leber den Hauptmetastasierungsort darstellen, z.B. im Falle von Brustkrebs oder bei Tumoren des Magen-Darm-Traktes. Die Leber kann aber auch das einzige Metastasierungsorgan darstellen, z.B. beim operierten Dickdarmkrebs, der später zur Lebermetastasierung führt. Demgegenüber sind primäre Leberzellkarzinome ausschließlich auf die Leber beschränkt.

Beispiel 2: Entzündliche Lebererkrankungen

Mit den Interferonen (Ifn) steht erstmals eine Substanzgruppe zur Verfügung, die bei der Behandlung der chronischen Virushepatitis erfolgversprechend ist. Der Einschluß von Ifn in Liposomen und deren quantitativer Transport in die erkrankte Leber läßt eine erhebliche Reduktion von Ifn-spezifischen Nebenwirkungen erwarten. Außerdem dürften die Zeitintervalle für die Applikation von Ifn wesentlich verlängert werden, möglicherweise kann von einer 4 x wöchentlichen auf eine 1 x wöchentliche Applikation zurückgegangen werden.

Aufgabenstellung der vorliegenden Erfindung ist deshalb die Bereitstellung von Liposomen, mit denen das vorstehend angesprochene Problem eines organspezifischen Wirkstofftransportes gelöst werden kann und mit denen es möglich ist, eine Verbesserung des therapeutischen Index der in den Liposomen eingeschlossenen Wirkstoffe zu erreichen.

Diese Aufgabe wird mit dem Gegenstand der vorliegenden Erfindung gelöst.

Gegenstand der vorliegenden Erfindung sind neue Liposomenstrukuren, die dadurch gekennzeichnet sind, daß sie mindestens 1 Mol-% einer eine positive Überschußladung aufweisenden Verbindung der allge-

meinen Formel (I) enthalten

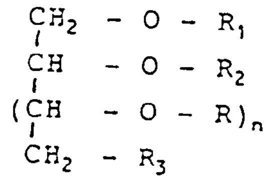

(I)

in der

$R_1$ Alkoyl oder Alkyl mit je 14 bis 18 C-Atomen, Oleoyl oder Oleyl bedeutet,

$R_2$ die PNN-Gruppe

PNN =

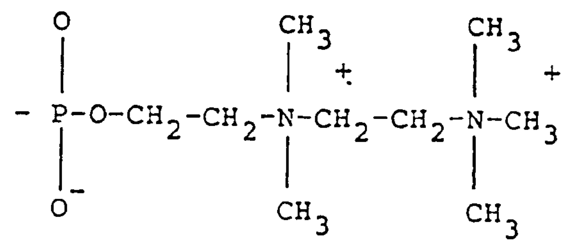

bedeutet,

$R_3$ = $-O-R_1$, -O-PNN, -O-Gly, $NH_3^+$, $NH_2CH_3^+$, $NH(CH_3)_2^+$ oder $N(CH_3)_3^+$ bedeutet,

R eine der für $R_1$, $R_2$ oder $R_3$ angegebenen Bedeutungen besitzt, und

n eine ganze Zahl von 0 bis 3, und vorzugsweise 0 bedeutet, mit der Maßgabe, daß das Molekül eine der genannten Gruppen mit positiver Ladung enthält.

Aus der DE-A-27 52 553 sind Phospholipidartige Verbindungen und Verfahren zu ihrer Herstellung bekannt, die eine Struktur aufweisen, die mit der vorstehend genannten Formel (I), worin einer der Reste R, $R_1$, $R_2$ oder $R_3$ PNN umfaßt, vergleichbar

Es wurde nun überraschenderweise gefunden, daß Liposomen aus Lipidgemischen, die wenigstens 3 Mol-% einer Verbindung der allgemeinen Formel (I) enthalten, ein organspezifisches Verhalten als Arzneistoffträger zeigen. Vorzugsweise enthalten die erfindungsgemäßen Liposomen 1 bis 30 Mol-%, und insbesondere 5 bis 15 Mol-% einer Verbindung der allgemeinen Formel (I), wobei die Liposomen auch Gemische aus einer oder mehreren Verbindungen der allgemeinen Formel (I) enthalten können.

Die nachstehende Tabelle 1 zeigt, daß für [$^3$H]-Inulin markierte erfindungsgemäße Liposomen die eine eine positive Überschußladung aufweisende Verbindung der allgemeinen Formel (I) enthalten, der Gehalt an [$^3$H]-Inulin im Blut bereits nach einer Stunde auf weniger als 1 % zurückgegangen ist. Ein ausserordentlich hoher Prozentsatz an [$^3$H]-Inulin findet sich dagegen in der Leber: nach 1 Stunde 67 %, nach 24 Stunden 54 % und nach 72 Stunden immer noch 45 %. In Organen wie Lunge, Niere, Herz und Gehirn kann Inulin nicht nachgewiesen werden. Deshalb sind diese Organe in Tabelle 1 nicht aufgeführt. Selbst in der Milz, die als Teil des phagozytierenden Systems Liposomen normalerweise gut aufnimmt, werden Liposomen überraschenderweise nicht gefunden. Dies wird mit der nachstehenden Tabelle 2 verdeutlicht, in der die [$^3$H]-Inulinaufnahmen für Blut, Leber und Milz pro g Frischgewicht dargestellt sind.

Tabelle 1

Vergleich der [$^3$H]-Inulinaufnahme nach Einschluß in Liposomen verschiedener Oberflächenladung und nach Gabe von freiem [$^3$H]-Inulin.

| Lipide (Mol/Mol) | Zeit (Std.) | Blut | Leber | Milz |
|---|---|---|---|---|
| | | (% der applizierten Menge) | | |
| PPGPC/Chol | 1 | 59 | 11 | 3 |
| 60 / 40 | 24 | 3 | 20 | 5 |
| | 72 | 0 | 17 | 2 |
| PPGPC/PPGPG/Chol | 1 | 64 | 7 | 1 |
| 50 / 10 / 40 | 24 | 8 | 16 | 1 |
| | 72 | 0 | 11 | 2 |
| PPGPC/ $N^+$/Chol | 1 | 1 | 67 | 2 |
| 50 /10/ 40 | 24 | 0 | 54 | 0 |
| | 72 | 0 | 45 | 0 |
| freies [$^3$H]-Inulin | 1 | 0.6 | 0 | 0 |
| | 24 | 0 | 0 | 0 |

Die Verteilung in den Organen ist in Prozent der applizierten Menge an Inulin angegeben. Für einen großen Dosisbereich (bis 1 mMol pro kg Körpergewicht) ist die Aufnahme an [$^3$H]-Inulin in NMR-I Mäusen fast linear. Freies Inulin hat den Blutkreislauf bereits nach 1 Stunde verlassen und wird vollständig über die Niere ausgeschieden. Die verschiedenen Abkürzungen bedeuten: Std. = Stunde; PPGPC = 1.2-Dipalmitoyl-sn-glycero-3-phosphocholin; Chol = Cholesterin; PPGPG = 1.2-Dipalmitoyl-sn-glycero-3-phospho-sn-1-glycerin, $Na^+$-Salz; $N^+$ = ein positives, zweikettiges Lipid aus der allgemeinen Formel (I).

Tabelle 2

Vergleich der [$^3$H]-Inulinaufnahme nach Einschluß in Liposomen in Prozent pro g Frischgewicht. Alle anderen Angaben entsprechen denen in Tabelle 1.

| Lipide (Mol/Mol) | Zeit (Std.) | Blut | Leber | Milz |
|---|---|---|---|---|
| | | (% der appl. Menge pro g Frischgew.) | | |
| PPGPC/Chol | 1 | 26 | 8 | 19 |
| 60 / 40 | 24 | 2 | 15 | 38 |
| PPGPC/PPGPG/Chol | 1 | 35 | 6 | 11 |
| 50 / 10 / 40 | 24 | 4 | 13 | 18 |
| PPGPC/$N^+$/Chol | 1 | 0 | 37 | 11 |
| 50 /10/40 | 24 | 0 | 31 | 0 |
| freies [$^3$H]-Inulin | 1 | 0.6 | 0 | 0 |
| | 24 | 0 | 0 | 0 |

Aus den in den vorstehend angegebenen Tabellen 1 und 2 angegebenen Werten ist ersichtlich, daß mit den erfindungsgemäßen Liposomen aus zweikettigen lipophilen Strukturen, die eine eine positive Überschußladung aufweisende Verbindung der allgemeinen Formel (I) enthalten, eine ausschließliche Anreicherung des Wirkstoffs in der Leber möglich ist. Diese überraschende Tatsache kann zur Therapie von Lebererkrankungen ausgenutzt werden.

Weiterer Gegenstand der vorliegenden Erfindung ist deshalb auch eine Arzneimittelzubereitung zur Behandlung von Lebererkrankungen, enthaltend einen oder mehrere Wirkstoffe gegen Erkrankungen in der Leber, die in Liposomen eingeschlossen sind, dadurch gekennzeichnet, daß die Liposomen mindestens 1 Mol-%, vorzugsweise 1 bis 30 Mol-%, und insbesondere 5 bis 15 Mol-%, an eine positive Überschußladung aufweisenden Verbindungen der vorstehenden allgemeinen Formel (I) enthalten, worin $R_1$, $R_2$, $R_3$, R und n die vorstehend angegebenen Bedeutungen besitzen. Die eingeschlossene Wirkstoffmenge ist nach oben begrenzt durch die Löslichkeit des Wirkstoffs in Wasser für wasserlösliche Pharmaka.

Weiterer Gegenstand ist auch die Verwendung einer Verbindung der allgemeinen Formel (I) zur Herstellung eines Arzneimittels zur Behandlung von Lebererkrankungen, das einen oder mehrere Wirkstoffe gegen Erkrankungen im Bereich der Leber in Liposomen eingeschlossen enthält, und worin die Liposomen mindestens 1 Mol-% einer Verbindung der Formel (I) enthalten.

Die erfindungsgemäßen Lipide der allgemeinen Formel (I), die zur Herstellung von erfindungsgemäßen Liposomen zur Anreicherung in der Leber verwendet werden können, sind:

Phospholipidartige Strukturen, die den natürlicherweise nicht vorkommenden Rest PNN enthalten .

Bevorzugte Lipide sind:

1,2-MM-sn-G-3-PNN, 1,2-PP-sn-G-3-PNN, 1,2-SS-sn-G-3-PNN, 1,2-TeTe-sn-G-3-PNN, 1,2-HeHe-sn-G-3-PNN und 2,2-OcOc-sn-G-3-PNN; oder die entsprechenden Phospholipide auf der Basis von 1,3-Diacyl- oder 1,3-Dialkylglycerinen als Ausgangsprodukten.

Die erfindungsgemäß verwendeten Lipide können z.B. nach den für derartige Verbindungen in der DE-A-27 52 553 angegebenen Verfahren hergestellt werden.

Bei den vorstehend angegebenen Herstellungsverfahren für die Verbindungen der allgemeinen Formel (I) arbeitet man vorzugsweise mit den in den Beispielen zur Herstellung erfindungsgemäßer Verbindungen angegebenen allgemeinen Verfahrensweisen und unter den dort angegebenen Reaktionsbedingungen.

Zur Herstellung der erfindungsgemäßen Liposomen mit positiver Überschußladung werden die einzelnen Liposomen-Komponenten, z.B. PPGPC, Chol und $N^+$, wobei $N^+$ eine oder mehrere erfindungsgemäße Lipide der allgemeinen Formel (I) darstellt, in einem geeigneten Lösungsmittel, vorzugsweise unter Erwärmen, gelöst, um eine homogene Vermischung der Komponenten zu erreichen. Das Lösungsmittel wird im Vakuum entfernt und der feinverteilte Lipidfilm wird mit einer wäßrigen Pufferlösung (als wäßrige Pufferlösung können alle physiologisch verwendbaren Lösungen eingesetzt werden) versetzt. Anschließend wird das Gemisch unter milder Agitation ca. 1 Stunde lang auf einer Temperatur gehalten, die im allgemeinen etwa 5°C über der Hauptumwandlungstemperatur der Lipide liegt, z.B. bei ca. 50°C.

Die vorgetemperte Lipidsuspension wird dann in die Druckzelle einer French-Press (Firma Amico, Silver Spring, USA) überführt. Die French-Press besteht aus einer hydraulischen Presse und einer Standarddruckzelle aus Stahl mit einem maximalen Füllvolumen von 40 ml. Nach Verschließen der Druckzelle wird der Druck auf 20 000 psi erhöht und die Liposomendispersion unter konstantem Druck durch einen schmalen Auslaß gepreßt. Der Vorgang wird mindestens dreimal wiederholt. Nach Zentrifugation der Liposomendispersion (Sorvall RC-5B: 5°C, 30 Minuten bei 27 000 g) wird der Überstand vom Sediment abgehoben. Er enthält die Liposomen, die nun für die verschiedenen Verwendungen und Untersuchungen, z.B. zur Herstellung einer erfindungsgemäßen Arzneimittelzubereitung (wirkstoffhaltige Liposomen) zur Verfügung stehen. Die erfindungsgemäßen Liposomen können ebenso nach anderen Verfahren hergestellt werden.

Gegenstand der vorliegenden Erfindung ist deshalb auch ein Verfahren zur Herstellung von erfindungsgemäßen Liposomen, die mindestens 3 Mol-% einer eine positive Überschußladung aufweisenden Verbindung der allgemeinen Formel (I), worin $R_1$, $R_2$, $R_3$, R und n die vorstehend angegebenen Bedeutungen besitzen, enthalten, und das dadurch gekennzeichnet ist, daß man mindestens 3 Mol-% einer Verbindung der allgemeinen Formel (I) zusammen mit den weiteren Liposomen-Komponenten in einer Menge, die zusammen mit der Verbindung der allgemeinen Formel (I) 100 Mol-% ergibt.

Die erfindungsgemäßen Verbindungen der Formel (I) werden dabei vorzugsweise in einer Menge von 1 bis 30 Mol-%, und insbesondere von 5 bis 15 Mol-% (Gesamtliposomen = 100 Mol-%) eingesetzt.

Zur Herstellung einer Arzneimittelzubereitung, die einen oder mehrere Wirkstoffe, die in den erfindungsgemäßen Liposomen eingeschlossen sind, enthalten, (Herstellung der wirkstoffhaltigen Liposomen) wird wie folgt verfahren:

Zum Einschluß wasserunlöslicher Substanzen wird der Wirkstoff mit den Lipiden in Methylenchlorid oder Chloroform gelöst; daran anschließend wird nach dem vorstehend für die Herstellung der erfindungsgemäßen Leerliposomen beschriebenen Verfahren gearbeitet.

Zum Einschluß wasserlöslicher Substanzen wird, wie vorstehend zur Herstellung der Leerliposomen beschrieben, der Lipidfilm mit einer Pufferlösung versetzt, die jetzt aber den wasserlöslichen Wirkstoff enthält. Anschließend wird, wie bei der Herstellung der Leerliposomen beschrieben, weiter verfahren. Der Überstand nach der Zentrifugation enthält zusätzlich zu den gefüllten Liposomen auch den nicht eingeschlossenen wasserlöslichen Wirkstoff. Dieser freie Wirkstoffanteil kann von dem in Liposomen eingeschlossenen Anteil durch Gelchromatographie abgetrennt werden (vgl. Liposomes: From physical structure to therapeutic application (1981), 1.c.). Vorzugsweise wird eine Aufkonzentrierung der Liposomen durch Ultrafiltration vorgenommen (Firma Amicon oder Sartorius). Zweckmäßigerweise wird vor der Verwendung der Liposomen eine Sterilfiltration mit Membranfiltern durchgeführt (Firma Sartorius, Porenweiten 0,2 $\mu$m).

Gegenstand der vorliegenden Erfindung ist deshalb auch ein Verfahren zur Herstellung einer Arzneimittelzubereitung, die einen oder mehrere Wirkstoffe enthält, die in die erfindungsgemäßen Liposomen eingeschlossen sind, das dadurch gekennzeichnet ist, daß man nach dem Verfahren zur Herstellung der erfindungsgemäßen Liposomen, die mindestens 3 Mol-% einer Verbindung der allgemeinen Formel (I) enthalten, arbeitet, und zum Einschluß wasserunlöslicher Wirkstoffe den Wirkstoff zusammen mit den Lipiden löst und zum Einschluß wasserlöslicher Wirkstoffe den Lipidfilm mit einer wäßrigen Pufferlösung versetzt, die den wasserlöslichen Wirkstoff enthält.

Vorzugsweise sollen die Wirkstoffe in die Leber transportiert werden. Es werden vorzugsweise ein oder mehrere Wirkstoffe ausgewählt, z.B. aus der Gruppe der Zytostatika (Hexadecyl-phosphocholin, 1-Octadecyl-2-methyl-rac-glycero-3-phosphocholin, 5-Fluorourazil, Epirubicin, Adriamycin, cis-Platinkomplexe, Novantron), aus der Klasse der immunmodulierenden Substanzen (Interferon $\alpha$, MAF = macrophage activating

factor), antimykotisch wirksame Substanzen (Amphotericin B) und Wirkstoffe gegen Protozoenerkrankungen (Malaria, Trypanosomen- und Leishmanien-Infektionen).

Die nachfolgenden Beispiele sollen die Erfindung näher erläutern, ohne sie darauf zu beschränken.

Wenn nicht anders angegeben, beziehen sich Mengen- und Prozentangaben auf das Gewicht, Temperaturangaben auf die Celsius-Skala.

Beispiele

Beispiele zur Herstellung erfindungsgemäßer Verbindungen der Lipidgruppe A:
Allgemeine Verfahrensweise:

Zur Herstellung der Verbindungen geht man so vor, daß man

a) eine Verbindung der allgemeinen Formel (I), worin $R_3$ eine OH-Gruppe bedeutet (nachfolgend als Verbindung der allgemeinen Formel (II) bezeichnet) mit $POCl_3$ in an sich bekannter Weise umsetzt,

b) das Reaktionsprodukt von Stufe a) in an sich bekannter Weise mit einer Verbindung der allgemeinen Formel (III)

$$HO - Alk - Hal \qquad (III)$$

in der Hal Cl, Br oder J bedeutet, umsetzt,

c) das Reaktionsprodukt von Stufe b) mit einer Verbindung der allgemeinen Formel (IV)

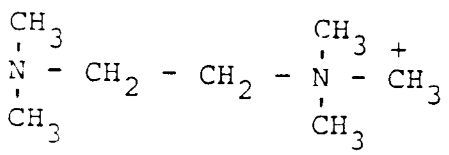

(IV)

umsetzt.

Unter den Verbindungen der allgemeinen Formel (III) werden die bromhaltigen bevorzugt.

Die Umsetzung von Phosphoroxychlorid mit der Verbindung der allgemeinen Formel (II) in Stufe a) wird vorzugsweise in einem inerten organischen Lösungsmittel durchgeführt. Beispiele für geeignete Lösungsmittel sind halogenierte Kohlenwasserstoffe, wie Chloroform und Tetrachlorkohlenstoff, aromatische Kohlenwasserstoffe, wie Benzol oder Toluol und aliphatische Kohlenwasserstoffe, wie Petrolether und dergleichen. Ebenfalls geeignet sind cyclische organische Lösungsmittel, wie Tetrahydrofuran. Bevorzugt werden Trichlorethylen und Tetrahydrofuran, da hierbei die entstehenden Salze, wie Triethylamin-hydrochlorid, eine sehr geringe Löslichkeit aufweisen und daher ausfallen und durch Abfiltrieren leicht abgetrennt werden können. Die Umsetzung sollte möglichst unter Feuchtigkeitsausschluß durchgeführt werden. Geeignete Temperaturen liegen im Bereich von -10 bis 50°C, vorzugsweise zwischen 10 und 30°C. Je nach den verwendeten Substanzen und Lösungsmitteln kommen jedoch in manchen Fällen auch darüber- und darunterliegende Temperaturen zur Anwendung.

Die Umsetzung wird vorzugsweise in Anwesenheit einer inerten organischen Base, wie Triethylamin, Pyridin oder Chinolin durchgeführt.

Zweckmäßig löst man das Phosphoroxychlorid in dem inerten Lösungsmittel und gibt die Base zu. Dann wird die Verbindung der allgemeinen Formel (II), zweckmäßig ebenfalls in einem inerten Lösungsmittel gelöst, zugesetzt. Dies erfolgt vorzugsweise durch Eintropfen unter Rühren. Da die Umsetzungen glatt und eindeutig verlaufen, kann die Temperatur von Fall zu Fall so gewählt werden, daß die Umsetzung direkt nach dem Eintropfen beendigt ist, was durch dünnschichtchromatographische Untersuchung leicht festgestellt werden kann.

Die Stufe b) läuft glatt ab, wenn man das Produkt der Stufe a) mit der Verbindung der allgemeinen Formel (III) mischt. Vorzugsweise versetzt man hierzu das Reaktionsgemisch mit einer Lösung der Verbindung der allgemeinen Formel (III) in Gegenwart einer organischen Base, wie Triethylamin. Bevorzugt erfolgt die Umsetzung bei Temperaturen zwischen 20 und 60°C unter Verwendung von Tetrahydrofuran als Lösungsmittel. Je nach den angewandten Bedingungen beträgt die Reaktionsdauer im allgemeinen zwischen etwa 20 und 120 Minuten.

Unter den bevorzugten Bedingungen fällt das Hydrohalogenid der Base aus und wird entfernt. Zur Erzielung bester Ausbeuten wird das Hydrochlorid nachgewaschen und die Waschflüssigkeit der Reaktionslösung wieder zugegeben. Dann wird das Lösungsmittel entfernt. Der Rückstand wird dann gegebenenfalls in Tetrahydrofuran gelöst und mit einer schwach alkalischen wäßrigen Lösung, beispielsweise Natriumbicarbonat in Wasser, hydrolysiert, wobei vorzugsweise der pH-Wert zwischen 5 und 7 gehalten wird. Dann wird mit einem organischen Lösungsmittel, wie Diisopropylether oder Chloroform, extrahiert. Man erhält so die Natriumsalze

der Alkylphosphatidsäuren, die sich gut umkristallisieren lassen.

Die Reaktion in Stufe c), also die Umsetzung des Produkts von Stufe b) mit der Aminobase, erfolgt ebenfalls im allgemeinen in polaren Lösungsmitteln, wie beispielsweise Chloroform, primäre, sekundäre oder tertiäre Alkohole, Dimethylformamid, Acetonitril, Nitromethan oder Wasser bzw. Gemischen davon. Für die Umsetzung kommen je nach der Empfindlichkeit der verwendeten Ausgangssubstanzen alle Temperaturen im Bereich zwischen dem Festpunkt und dem Siedepunkt des verwendeten Lösungsmittels bzw. Lösungsmittelgemisches in Betracht. Bevorzugt erfolgt die Umsetzung bei Temperaturen zwischen Zimmertemperatur und dem Siedepunkt des Lösungsmittels. So sind bei 50°C die Umsetzungen in der Regel nach 2 bis 8 Stunden beendet. Das Reaktionsprodukt wird anschließend isoliert und kann umkristallisiert werden. Auch eine chromatographische Reinigung ist möglich. Die Ausbeuten liegen im allgemeinen über 50 % der Theorie, bezogen auf Diglycerid als Ausgangsprodukt.

Die erzielten hohen Ausbeuten an gewünschtem Produkt sind überraschend, da bei den vielen funktionellen Gruppen in den Reaktionspartnern das glatte Ablaufen der Reaktion in der gewünschten Richtung nicht vorhersehbar war.

Die vorstehend angegebenen Verfahrensstufen a), b) und c) werden vorzugsweise wie folgt durchgeführt:

<u>Stufe a)</u>

10 ml Trichlorethylen und 6,6 g $POCl_3$ (0,044 Mol) werden in einem Eisbad bei 0 bis 5°C mit 0,04 Mol eines Alkohols der allgemeinen Formel (II) - gelöst in 40 ml Trichlorethylen und 9 g Triethylamin - versetzt. Handelt es sich bei dem Alkohol um ein Diacylglycerin, so wird zur Vermeidung von Acylwanderung das Acylierungsgemisch nacheinander mit 9 g Triethylamin in 10ml Trichlorethylen und dann sofort mit dem Diacylglycerin in 30 ml Trichlorethylen versetzt. Man verwendet 25 ml Toluol zum Nachwaschen. Man ersetzt das Eisbad durch ein Wasserbad bei 20°C. Die Reaktion ist schon nach 20 Minuten bei 20°C beendet.

<u>Stube b)</u>

Bei 20°C gibt man zu dem Reaktionsgemisch von Stufe a) 0,048 Mol Verbindung (III), z.B. gelöst in 75 ml Tetrahydrofuran und 13 g Triethylamin und verwendet 25 ml Tetrahydrofuran zum Nachwaschen. Nach 20 Minuten bei 35°C ist die Reaktion abgelaufen. Man filtriert, wäscht in 50 ml Toluol nach und engt ein. Die Hydrolyse erfolgt durch Zusatz von nacheinander 30 ml Eiswasser, nach 2 Minuten 30 ml 1 M Natriumacetat und nach weiteren 2 Minuten 90 ml Tetrahydrofuran. Nach 12 Stunden ist die Hydrolyse beendet. Es entsteht praktisch nur ein Produkt.

<u>Stufe c)</u>

B. 0,04 Mol Produkt von Stufe b) (β-Bromethylester) werden in 90 ml $CHCl_3$ gelöst, mit 150 ml isopropanol sowie mit 200 ml 40 % N,N,N,N-Tetramethylethylendiamin in Wasser versetzt.

Mit dem vorstehend beschriebenen Verfahren werden die nachfolgenden Verbindungen Nr. 1 bis 8 erhalten:

1) 1,2-Dimyristoyl-sn-glycero-3-phospho-(N,N-dimethyl-N-[N',N',N'-trimethyl-ethylammonio])-ethylammoniumchlorid, $C_{40}H_{82}ClN_2O_8P$ (785.5)
Nachweis durch DC-Vergleich mit dem 1,2-Dipalmitoyl-derivat (siehe Substanz 2)

2) 1,2-Dipalmitoyl-sn-glycero-3-phospho-(N,N-dimethyl-N-[N',N',N'-trimethyl-ethylammonio])-ethylammoniumchlorid, $C_{44}H_{90}ClN_2O_8P$ (841.6)

| | | | | |
|---|---|---|---|---|
| ber. (%): | C, 62.79; | H, 10.78; | N, 3.33; | P. 3.68 |
| gef. (%): | 62.54; | 10.71; | 3.21; | 3.67 |

3) 1,2-Distearoyl-sn-glycero-3-phospho-(N,N-dimethyl-N-[N',N',N'-trimethyl-ethylammonio])-ethylammoniumchlorid $C_{48}H_{98}ClN_2O_8P$ (897.8)
Nachweis durch DC-Vergleich mit dem 1,2-Dipalmitoylderivat (siehe Substanz 2)

4) 1,2-Dipalmitoyl-sn-glycero-3-phospho-(N,N-dimethyl-N-[N',N'-dimethyl-ethylamino])-ethylammonium $C_{43}H_{87}N_2O_8$ (791.2)

ber. (%): C, 65.28; H, 11.09; N, 3.54; P, 3.92
gef. (%): 65.11; 11.02 3.47; 3.88

5) 1,2-Dihexadecyl-sn-glycero-3-phospho-(N,N-dimethyl-N-[N',N',N'-trimethyl-ethylammonio]-ethylammoniumchlorid $C_{44}H_{94}ClN_2O_6P$ (813.7)
Nachweis durch DC-Vergleich mit dem 1,2-Dipalmitoylderivat (siehe Substanz 2)
6) 1,2-Dioleyl-rac-glycero-3-phospho-(N,N-dimethyl-N-[N',N',N'-trimethyl-ethylamino]-ethylammoniumchlorid $C_{48}H_{98}ClN_2O_6P$ (865.8)

ber. (%): C, 66.59; H, 11.41; N, 3.24; P, 3.58
gef. (%): 66.41; 11.35; 3.19; 3.45

7) 1,3-Dihexadecyl-glycero-2-phospho-(N,N-dimethyl-N-[N',N',N'-trimethyl-ethylammonio]-ethylammoniumchlorid $C_{44}H_{94}ClN_2O_6P$ (813.7)

ber. (%): C, 64.95; H, 11.65; N, 3.44; P, 3.81
gef. (%): 64.79; 11.59; 3.42; 3.69

8) 1,3-Dioleoyl-rac-glycero-3-phospho-(N,N-dimethyl-N-[N',N',N'-trimethylamino]-ethylammoniumchlorid

ber. (%): C, 66.59; H, 11.41; N, 3.24; P, 3.58
gef. (%): 66.55; 11.40; 3.11; 3.55

Herstellung von erfindungsgemäßen Leerliposomen:

Es werden die in Tabelle 1 angegebenen erfindungsgemäßen Liposomen PPGPC/$N^+$/Chol (50/10/40) hergestellt.

PPGPC (5 mMol), 1,2-Dihexadecyl-rac-glycero-3-phospho-(N,N-dimethyl-N-[N',N',N'-trimethylethylamino])-ethyl-ammoniumchlorid (1 mMol) und Cholesterin (4 mMol) werden in 50 ml $CH_2Cl_2$ oder 50 ml $CHCl_3$ unter Erwärmen gelöst, um eine homogene Vermischung der drei Komponenten zu erreichen. Das Lösungsmittel wird im Vakuum entfernt und der feinverteilte Lipidfilm mit 250 ml wäßriger Pufferlösung (es.können alle physiologisch verwendbaren Lösungen eingesetzt werden) versetzt. Anschließend hält man das Gemisch unter langsamer Rotation 60 Minuten bei 50°C (im allgemeinen etwa 5°C über der Hauptumwandlungstemperatur der Lipide).

Die vorgetemperte Lipidsuspension wird nun in die Druckzelle einer French-Press (Firma Amico, Silver Spring, USA) überführt. Die French-Press besteht aus einer hydraulischen Presse und einer Standarddruckzelle aus Stahl mit einem maximalen Füllvolumen von 40 ml. Nach Verschließen der Druckzelle wird der Druck auf 20 000 psi erhöht und die Liposomendispersion unter konstantem Druck durch einen schmalen Auslaß gepreßt. Der Vorgang wird mindestens dreimal wiederholt. Nach Zentrifugation der Liposomendispersion (Sorvall RC-5B: 5°C, 30 Minuten bei 27 000 g) wird der Überstand vom Sediment abgehoben. Er enthält die Liposomen, die nun für die verschiedenen Experimente zur Verfügung stehen.

Herstellung von erfindungsgemäßen Liposomen mit einem wasserlöslichen Wirkstoff, der zur Leber transportiert werden soll.

PPGPC (5 mMol), 1,2-Dihexadecyl-rac-glycero-3-phospho-(N,N-dimethyl-[N',N',N'-trimethylethylamino]-ethyl-ammoniumchlorid (1 mMol) und Cholesterin (4 mMol) werden in 50 ml $CH_2Cl_2$ oder 50 ml $CHCl_3$ unter Erwärmen gelöst, um eine homogene Vermischung der drei Komponenten zu erreichen. Das Lösungsmittel wird im Vakuum entfernt und der feinverteilte Lipidfilm mit 250 ml wäßriger Pufferlösung versetzt (es können alle physiologisch verwendbaren Lösungen eingesetzt werden). Anschließend hält man das Gemisch unter langsamer Rotation 60 Minuten bei 50°C (im allgemeinen etwa 5°C über der Hauptumwandlungstemperatur der Lipide). Die vorgetemperte Lipidsuspension wird nun in die Druckzelle einer French-Press (Fa. Amico, Silver Spring, USA) überführt. Die French-Press besteht aus einer hydraulischen Presse und einer Standarddruckzelle aus Stahl mit einem maximalen Füllvolumen von 40 ml. Nach Verschließen der Druckzelle wird der Druck auf 20 000 psi erhöht und die Liposomendispersion unter konstantem Druck durch einen schmalen Auslaß gepreßt. Der Vorgang wird mindestens dreimal wiederholt. Nach Zentrifugieren der

Liposomendispersion (Sorvall RC-5B; 5°C, 30 min bei 27 000 g) wird der Überstand vom Sediment abgehoben. Er enthält die Liposomen, die nun für die verschiedenen Experimente zur Verfügung stehen.

Zu dieser Lipidsuspension werden 50 ml einer 0,9%igen Lösung von Natriumchlorid gegeben, die MAF (Makrophage Activating Factor) in einer Proteinkonzentration von 70 µg/ml enthält. Dieses Gemisch wird analog zur Herstellung der Leerliposomen in der Druckzelle behandelt. Nach der anschließenden Zentrifugation wird der Überstand vom Sediment abgehoben und einer Gelchromatographie an Sepharose Cl-4B (LKB-Pharmacia, Gelbettlänge 70 cm) unterworfen. Dadurch wird nicht in die erfindungsgemäßen Liposomen eingeschlossener MAF von eingeschlossenem MAF sauber abgetrennt. Letzteres benötigt aufgrund der Größe der Liposomen ein deutlich geringeres Elutionsvolumen als nicht eingeschlossenes MAF (MW 30 000). Die Liposomen werden zur Verwendung aufkonzentriert und steril filtriert.

## Patentansprüche

### Patentansprüche für folgende Vertragsstaaten : AT, BE, CH, LI, DE, DK, FR, GB, IT, LU, NL, SE

1. Liposomen,
   **dadurch gekennzeichnet,**
   daß sie mindestens 1 Mol-% einer eine positive Überschußladung ausweisenden Verbindung der allgemeinen Formel (I) enthalten

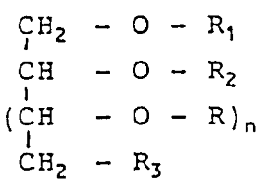

(I)

in der
$R_1$ Alkoyl oder Alkyl mit je 14 bis 18 C-Atomen, Oleoyl oder Oleyl bedeutet,
$R_2$ die PNN-Gruppe

PNN =

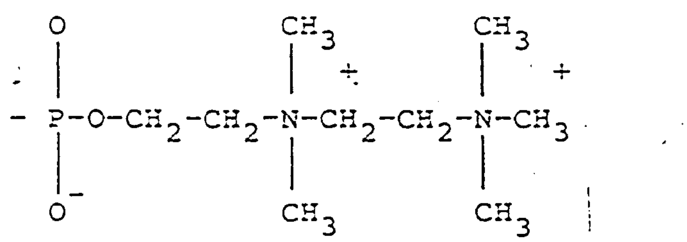

bedeutet,
$R_3$ = -O-$R_1$, -O-PNN, -O-Gly, $NH_3^+$, $NH_2CH_3^+$, $NH(CH_3)_2^+$ oder $N(CH_3)_3^+$ bedeutet,
R eine der für $R_1$, $R_2$ oder $R_3$ angegebenen Bedeutungen besitzt, und
n eine ganze Zahl von 0 bis 3 bedeutet, mit der Maßgabe, daß das Molekül eine der genannten Gruppen mit positiver Ladung enthält.

2. Liposomen nach Anspruch 1,
   **dadurch gekennzeichnet,**
   daß in Formel (I) n 0 ist.

3. Liposomen nach Anspruch 1 oder 2,
   **dadurch gekennzeichnet,**
   daß sie 1 bis 30 Mol-% einer Verbindung der Formel (I) enthalten.

4. Liposomen nach einem der Ansprüche 1 bis 3,
   **dadurch gekennzeichnet,**
   daß sie 5 bis 15 Mol-% einer Verbindung der Formel (I) enthalten.

5. Arzneimittelzubereitung zur Behandlung von Lebererkrankungen, enthaltend einen oder mehrere Wirkstoffe, die zur Leber transportiert werden sollen und in Liposomen eingeschlossen sind,
**dadurch gekennzeichnet,**
daß die Liposomen mindestens 1 Mol-% an eine positive Überschußladung aufweisenden Verbindungen der allgemeinen Formel (I) nach Anspruch 1 enthalten, worin $R_1$, $R_2$, $R_3$, R und n die in Anspruch 1 angegebene Bedeutung besitzen.

6. Arzneimittelzubereitung nach Anspruch 5,
**dadurch gekennzeichnet,**
daß in Formel (I) n 0 ist.

7. Arzneimittelzubereitung nach Anspruch 5 oder 6,
**dadurch gekennzeichnet,**
daß die Liposomen 1 bis 30 Mol-% einer Verbindung der Formel (I) enthalten.

8. Arzneimittelzubereitung nach einem der Ansprüche 5 - 7,
**dadurch gekennzeichnet,**
daß die Liposomen 5 bis 15 Mol-% einer Verbindung der Formel (I) enthalten.

9. Verwendung einer Verbindung der in Anspruch 1 angegebenen allgemeinen Formel (I), worin $R_1$, $R_2$, $R_3$, R und n die in Anspruch 1 oder 2 angegebene Bedeutung besitzen, zur Herstellung eines Arzneimittels zur Behandlung von Erkrankungen im Bereich der Leber.

10. Verfahren zur Herstellung von Liposomen nach einem der Ansprüche 1 bis 4,
**dadurch gekennzeichnet,**
daß man mindestens 1 Mol-% einer Verbindung der allgemeinen Formel (I) zusammen mit den weiteren Liposomen-Komponenten in einer Menge, die zusammen mit der Verbindung der allgemeinen Formel (I) 100 Mol-% ergibt, in eine Lipidsuspension überführt, die Lipidsuspension vortempert und die so vorgetemperte Lipidsuspension dann durch Pressen und Zentrifugation in an sich bekannter Weise in die Liposomen überführt.

11. Verfahren zur Herstellung einer Arzneimittelzubereitung nach einem der Ansprüche 5 bis 8,
**dadurch gekennzeichnet,**
daß man nach dem Verfahren gemäß Anspruch 10 zur Herstellung der erfindungsgemäßen Liposomen, die mindestens 1 Mol-% einer Verbindung der allgemeinen Formel (I) enthalten, arbeitet, und zum Einschluß wasserunlöslicher Wirkstoffe den Wirkstoff zusammen mit den Lipiden löst, und zum Einschluß wasserlöslicher Wirkstoffe den Lipidfilm mit einer wäßrigen Pufferlösung versetzt, die den wasserlöslichen Wirkstoff enthält.

## Patentansprüche für folgenden Vertragsstaat : GR

1. Liposomen,
**dadurch gekennzeichnet,**
daß sie mindestens 1 Mol-% einer eine positive Überschußladung ausweisenden Verbindung der allgemeinen Formel (I) enthalten

$$\begin{array}{l} CH_2 - O - R_1 \\ | \\ CH - O - R_2 \\ | \\ (CH - O - R)_n \\ | \\ CH_2 - R_3 \end{array} \qquad (I)$$

in der
$R_1$ Alkoyl oder Alkyl mit je 14 bis 18 C-Atomen, Oleoyl oder Oleyl bedeutet,
$R_2$ die PNN-Gruppe

PNN = 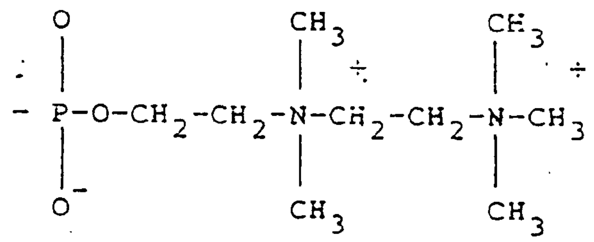

bedeutet,
$R_3$ = -O-$R_1$, -O-PNN, -O-Gly, $NH_3^+$, $NH_2CH_3^+$, $NH(CH_3)_2^+$ oder $N(CH_3)_3^+$ bedeutet,
R eine der für $R_1$, $R_2$ oder $R_3$ angegebenen Bedeutungen besitzt, und
n eine ganze Zahl von 0 bis 3 bedeutet, mit der Maßgabe, daß das Molekül eine der genannten Gruppen mit positiver Ladung enthält.

2. Liposomen nach Anspruch 1,
**dadurch gekennzeichnet,**
daß in Formel (I) n 0 ist.

3. Liposomen nach Anspruch 1 oder 2,
**dadurch gekennzeichnet,**
daß sie 1 bis 30 Mol-% einer Verbindung der Formel (I) enthalten.

4. Liposomen nach einem der Ansprüche 1 bis 3,
**dadurch gekennzeichnet,**
daß sie 5 bis 15 Mol-% einer Verbindung der Formel (I) enthalten.

5. Verfahren zur Herstellung von Liposomen nach einem der Ansprüche 1 bis 4,
**dadurch gekennzeichnet,**
daß man mindestens 1 Mol-% einer Verbindung der allgemeinen Formel (I) zusammen mit den weiteren Liposomen-Komponenten in einer Menge, die zusammen mit der Verbindung der allgemeinen Formel (I) 100 Mol-% ergibt, in eine Lipidsuspension überführt, die Lipidsuspension vortempert und die so vorgetemperte Lipidsuspension dann durch Pressen und Zentrifugation in an sich bekannter Weise in die Liposomen überführt.

6. Verfahren zur Herstellung einer Arzneimittelzubereitung
**dadurch gekennzeichnet,**
daß man nach dem Verfahren gemäß Anspruch 5 zur Herstellung der erfindungsgemäßen Liposomen, die mindestens 1 Mol-% einer Verbindung der allgemeinen Formel (I) enthalten, arbeitet, und zum Einschluß wasserunlöslicher Wirkstoffe den Wirkstoff zusammen mit den Lipiden löst, und zum Einschluß wasserlöslicher Wirkstoffe den Lipidfilm mit einer wäßrigen Pufferlösung versetzt, die den wasserlöslichen Wirkstoff enthält.

7. Verfahren nach Anspruch 6,
**dadurch gekennzeichnet,**
daß in Formel (I) n 0 ist.

8. Verfahren nach Anspruch 6 oder 7,
**dadurch gekennzeichnet,**
daß man Liposomen, die 1 bis 30 Mol-% einer Verbindung der Formel (I) enthalten, verwendet.

9. Verfahren nach Anspruch 8,
**dadurch gekennzeichnet,**
daß man Liposomen, die 5 bis 15 Mol-% einer Verbindung der Formel (I) enthalten, verwendet.

10. Verwendung einer Verbindung der in Anspruch 1 angegebenen allgemeinen Formel (I), worin $R_1$, $R_2$, $R_3$, R und n die in Anspruch 1 oder 2 angegebene Bedeutung besitzen, zur Herstellung eines Arzneimittels zur Behandlung von Erkrankungen im Bereich der Leber.

**Patentansprüche für folgenden Vertragsstaat : ES**

1. Verfahren zur Herstellung von Liposomen, **dadurch gekennzeichnet,** daß man mindestens 1 Mol-% einer Verbindung der allgemeinen Formel (I)

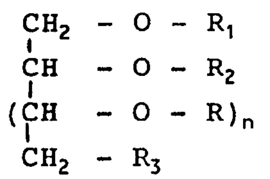

(I)

in der
$R_1$ Alkoyl oder Alkyl mit je 14 bis 18 C-Atomen, Oleoyl oder Oleyl bedeutet,
$R_2$ die PNN-Gruppe

PNN = 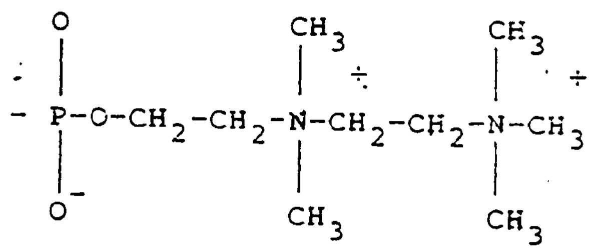

bedeutet,
$R_3$ = $-O-R_1$, -O-PNN, -O-Gly, $NH_3^+$, $NH_2CH_3^+$, $NH(CH_3)_2^+$ oder $N(CH_3)_3^+$ bedeutet,
R eine der für $R_1$ , $R_2$ oder $R_3$ angegebenen Bedeutungen besitzt, und
n eine ganze Zahl von 0 bis 3 bedeutet,
zusammen mit den weiteren Liposomen-Komponenten in einer Menge, die zusammen mit der Verbindung der allgemeinen Formel (I) 100 Mol-% ergibt, in eine Lipidsuspension überführt, die Lipidsuspension vortempert und die so vorgetemperte Lipidsuspension dann durch Pressen und Zentrifugation in an sich bekannter Weise in die Liposomen überführt.

2. Verfahren nach Anspruch 1, **dadurch gekennzeichnet,** daß in Formel (I) n 0 ist.

3. Verfahren nach Anspruch 1 oder 2, **dadurch gekennzeichnet,** daß man 1 bis 30 Mol-% einer Verbindung der Formel (I) einführt.

4. Verfahren nach einem der Ansprüche 1 bis 3, **dadurch gekennzeichnet,** daß man 5 bis 15 Mol-% einer Verbindung der Formel (I) einführt.

5. Verfahren zur Herstellung einer Arzneimittelzubereitung zur Behandlung von Lebererkrankungen, enthaltend einen oder mehrere Wirkstoffe, die zur Leber transportiert werden sollen und in Liposomen eingeschlossen sind, **dadurch gekennzeichnet,** daß man nach dem Verfahren gemäß Anspruch 1 zur Herstellung der Liposomen, die mindestens 1 Mol-% einer Verbindung der allgemeinen Formel (I) enthalten, arbeitet, und zum Einschluß wasserunlöslicher Wirkstoffe den Wirkstoff zusammen mit den Lipiden löst, und zum Einschluß wasser- löslicher Wirkstoffe den Lipidfilm mit einer wäßrigen Pufferlösung versetzt, die den wasserlöslichen Wirkstoff enthält.

6. Verfahren nach Anspruch 5,

**dadurch gekennzeichnet,**
daß in Formel (I) n 0 ist.

7. Verfahren nach Anspruch 5 oder 6,
**dadurch gekennzeichnet,**
daß die Liposomen 1 bis 30 Mol-% einer Verbindung der Formel (I) enthalten.

8. Verfahren nach einem der Ansprüche 5 - 7,
**dadurch gekennzeichnet,**
daß die Liposomen 5 bis 15 Mol-% einer Verbindung der Formel (I) enthalten.

## Claims

### Claims for the following Contracting States : AT, BE, CH, LI, DE, DK, FR, GB, IT, LU, NL, SE

1. Liposomes
**wherein**
they contain at least 1 mol-% of a compound of the general formula (I) with a positive excess charge

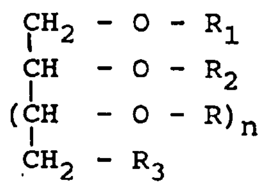

(I)

in which
$R_1$ denotes alkoyl or alkyl each with 14 to 18 C atoms, oleoyl or oleyl,
$R_2$ denotes the PNN group

PNN =

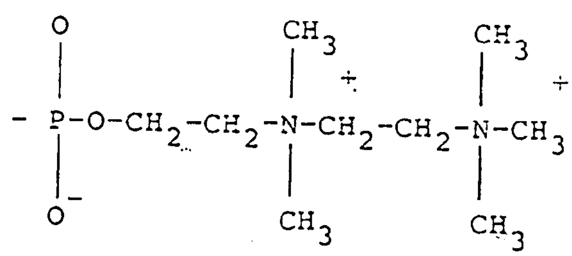

$R_3$ = -O-$R_1$, -O-PNN, -O-Gly, $NH_3^+$, $NH_2CH_3^+$, $NH(CH_3)_2^+$ or $N(CH_3)_3^+$,
R has one of the meanings stated for $R_1$, $R_2$ or $R_3$ and
n denotes an integer from 0 to 3 provided that the molecule contains one of the said groups with a positive charge.

2. Liposomes as claimed in claim 1,
**wherein**
n in formula (I) is 0.

3. Liposomes as claimed in claim 1 or 2
**wherein**
they contain 1 to 30 mol-% of a compound of formula (I).

4. Liposomes as claimed in one of the claims 1 to 3,
**wherein**
they contain 5 to 15 mol-% of a compound of formula (I).

5. Pharmaceutical preparation for the treatment of liver diseases containing one or several active substances which are to be transported to the liver and are enclosed in liposomes, **wherein** the liposomes contain at least 1 mol-% of a compound having the general formula (I) with positive excess charge as claimed in claim 1 in which $R_1$, $R_2$, $R_3$, R and n have the meaning stated in claim 1.

**6.** Pharmaceutical preparation as claimed in claim 5,
**wherein**
n in formula (I) is 0.

**7.** Pharmaceutical preparation as claimed in claim 5 or 6,
**wherein**
the liposomes contain 1 to 30 mol-% of a compound of formula (I).

**8.** Pharmaceutical preparation as claimed in one of the claims 5 - 7,
**wherein**
the liposomes contain 5 to 15 mol-% of a compound of formula (I).

**9.** Use of a compound having the general formula (I) stated in claim 1 in which $R_1$, $R_2$, $R_3$, R and n have the meanings stated in claim 1 or 2 for the production of a pharmaceutical agent for the treatment of diseases in the liver region.

**10.** Process for the production of liposomes as claimed in one of the claims 1 to 4,
**wherein**
at least 1 mol-% of a compound having the general formula (I) together with the other liposome components that are in an amount which together with the compound of the general formula (I) totals 100 mol-% are converted into a lipid suspension, the lipid suspension is pre-heated and the lipid suspension pre-heated in this manner is then converted in a well-known manner into liposomes by pressing and centrifuging.

**11.** Process for the production of a pharmaceutical preparation as claimed in one of the claims 5 to 8,
**wherein**
one uses the process as claimed in claim 10 for the production of the liposomes according to the invention which contain at least 1 mol-% of a compound having the general formula (I) and in order to enclose water-insoluble active substances, the active substance is dissolved together with the lipids and in order to enclose water-soluble active substances, an aqueous buffer solution containing the water-soluble active substance is added to the lipid film.

**Claims for the following Contracting State : GR**

**1.** Liposomes
**wherein**
they contain at least 1 mol-% of a compound of the general formula (I) with a positive excess charge

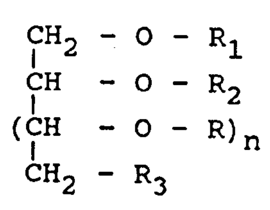

(I)

in which
$R_1$ denotes alkoyl or alkyl each with 14 to 18 C atoms, oleoyl or oleyl,
$R_2$ denotes the PNN group

PNN = 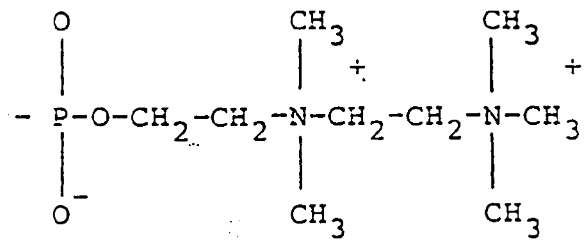

$R_3$ = -O-$R_1$, -O-PNN, -O-Gly, $NH_3^+$, $NH_2CH_3^+$, $NH(CH_3)_2^+$ or $N(CH_3)_3^+$,
R has one of the meanings stated for $R_1$, $R_2$ or $R_3$ and
n denotes an integer from 0 to 3 provided that the molecule contains one of the said groups with a positive charge.

2. Liposomes as claimed in claim 1,
**wherein**
n in formula (I) is 0.

3. Liposomes as claimed in claim 1 or 2
**wherein**
they contain 1 to 30 mol-% of a compound of formula (I).

4. Liposomes as claimed in one of the claims 1 to 3,
**wherein**
they contain 5 to 15 mol-% of a compound of formula (I).

5. Process for the production of liposomes as claimed in one of the claims 1 to 4,
**wherein**
at least 1 mol-% of a compound having the general formula (I) together with the other liposome components which are in an amount which together with the compound of the general formula (I) totals 100 mol-% are converted into a lipid suspension, the lipid suspension is pre-heated and the lipid suspension pre-heated in this manner is then converted in a well-known manner into liposomes by pressing and centrifuging.

6. Process for the production of a pharmaceutical preparation,
**wherein**
one uses the process as claimed in claim 5 for the production of the liposomes according to the invention which contain at least 1 mol-% of a compound having the general formula (I) and in order to enclose water-insoluble active substances, the active substance is dissolved together with the lipids and in order to enclose water-soluble active substances, an aqueous buffer solution containing the water-soluble active substance is added to the lipid film.

7. Process as claimed in claim 6,
**wherein**
n in formula (I) is 0.

8. Process as claimed in claim 6 or 7,
**wherein**
liposomes are used which contain 1 to 30 mol-% of a compound of formula (I).

9. Process as claimed in claim 8,
**wherein**
liposomes are used which contain 5 to 15 mol-% of a compound of formula (I).

10. Use of a compound of the general formula (I) given in claim 1 in which $R_1$, $R_2$, $R_3$, R and n have the meanings stated in claim 1 or 2 for the production of a pharmaceutical agent for the treatment of diseases in the liver region.

**Claims for the following Contracting State : ES**

1. Process for the production of liposomes
**wherein**
at least 1 mol-% of a compound of the general formula (I)

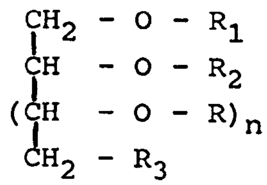

(I)

in which
$R_1$ denotes alkoyl or alkyl each with 14 to 18 C atoms, oleoyl or oleyl,
$R_2$ denotes the PNN group

PNN =

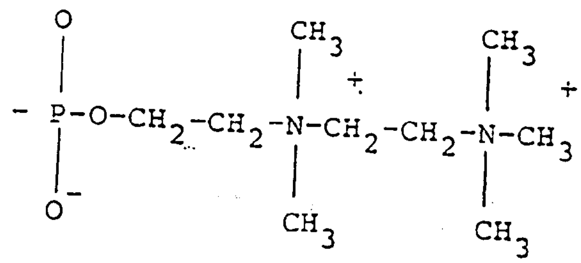

$R_3$ = $-O-R_1$, -O-PNN, -O-Gly, $NH_3^+$, $NH_2CH_3^+$, $NH(CH_3)_2^+$ or $N(CH_3)_3^+$,
R has one of the meanings stated for $R_1$, $R_2$ or $R_3$ and
n denotes an integer from 0 to 3,
together with the further liposome components which are in an amount which together with the compound of the general formula (I) totals 100 mol-% are converted into a lipid suspension, the lipid suspension is pre-heated and the lipid suspension pre-heated in this manner is then converted in a well-known manner into liposomes by pressing and centrifuging.

2. Process as claimed in claim 1,
**wherein**
n in formula (I) is 0.

3. Process as claimed in claim 1 or 2
**wherein**
1 to 30 mol-% of a compound of formula (I) is introduced.

4. Process as claimed in one of the claims 1 to 3,
**wherein**
5 to 15 mol-% of a compound of formula (I) is introduced.

5. Process for the production of a pharmaceutical preparation for the treatment of liver diseases containing one or several active substances which are to be transported to the liver and enclosed in liposomes,
**wherein**
one uses the process as claimed in claim 1 for the production of the liposomes which contain at least 1 mol-% of a compound having the general formula (I) and in order to enclose water-insoluble active substances, the active substance is dissolved together with the lipids and in order to enclose water-soluble active substances, an aqueous buffer solution containing the water-soluble active substance is added to the lipid film.

6. Process as claimed in claim 5,

**wherein**
n in formula (I) is 0.

7. Process as claimed in claim 5 or 6,
**wherein**
the liposomes contain 1 to 30 mol-% of a compound of formula (I).

8. Process as claimed in one of the claims 5 - 7,
**wherein**
the liposomes contain 5 to 15 mol-% of a compound of formula (I).

## Revendications

**Revendications pour les Etats contractants suivants : AT, BE, CH, LI, DE, DK, FR, GB, IT, LU, NL, SE**

1. Liposomes, caractérisés en ce qu'ils contiennent au moins 1 % en moles d'un composé présentant un excès de charge positive de formule générale (I)

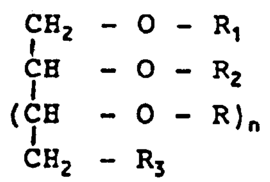

(I)

dans laquelle
$R_1$ représente un alcanoyle ou un alkyle ayant chacun 14 à 18 atomes de carbone, un oléoyle ou un oléyle,
$R_2$ représente le groupe PNN

PNN =

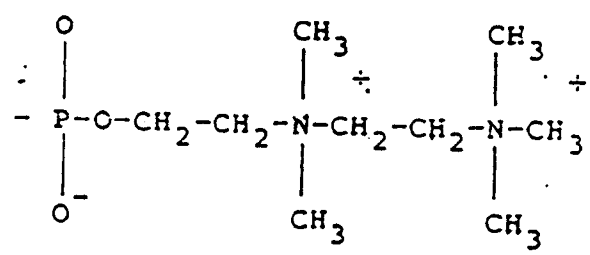

$R_3$ signifie -O-$R_1$, -O-PNN, -O-Gly, $NH_3^+$, $NH_2CH_3^+$, $NH(CH_3)_2^+$ ou $N(CH_3)_3^+$,
R possède l'une des significations données pour $R_1$, $R_2$ ou $R_3$, et
n représente un nombre entier de 0 à 3, sous réserve que la molécule contienne l'un des groupes à charge positive indiqués.

2. Liposomes selon la revendication 1, caractérisés en ce que, dans la formule (I), n est 0.

3. Liposomes selon la revendication 1 ou 2, caractérisés en ce qu'ils contiennent 1 à 30 % en moles d'un composé de formule (I).

4. Liposomes selon l'une des revendications 1 à 3, caractérisés en ce qu'ils contiennent 5 à 15 % en moles d'un composé de formule (I).

5. Composition de médicament pour le traitement de maladies du foie, contenant une ou plusieurs substances actives qui doivent être transportées vers le foie et qui sont incluses dans des liposomes, caractérisée en ce que les liposomes contiennent au moins 1 % en moles de composés présentant un excès de charge positive de formule générale (I) selon la revendication 1, dans laquelle $R_1$, $R_2$, $R_3$, R et n ont la signification donnée dans la revendication 1.

**6.** Composition de médicament selon la revendication 5, caractérisée en ce que, dans la formule (I), n est 0.

**7.** Composition de médicament selon la revendication 5 ou 6, caractérisée en ce que les liposomes contiennent 1 à 30 % en moles d'un composé de formule (I).

**8.** Composition de médicament selon l'une des revendications 5 à 7, caractérisée en ce que les liposomes contiennent 5 à 15 % en moles d'un composé de formule (I).

**9.** Utilisation d'un composé ayant la formule générale (I) donnée dans la revendication 1, dans laquelle $R_1$, $R_2$, $R_3$, R et n ont la signification donnée dans la revendication 1 ou 2, pour la préparation d'un médicament pour le traitement de maladies dans le domaine du foie.

**10.** Procédé de préparation de liposomes selon l'une des revendications 1 à 4, caractérisé en ce que l'on transforme en une suspension de lipides au moins 1% en moles d'un composé de formule générale (I) avec les autres constituants des liposomes en une quantité qui donne 100 % en moles avec le composé de formule générale (I), on soumet la suspension de lipides à un traitement thermique préalable et on transforme ensuite la suspension de lipides ainsi prétraitée à la chaleur en liposomes de façon connue en soi par pressage et centrifugation.

**11.** Procédé de préparation d'une composition de médicament selon l'une des revendications 5 à 8, caractérisé en ce que l'on travaille suivant le procédé selon la revendication 10 pour préparer les liposomes selon l'invention qui contiennent au moins 1 % en moles d'un composé de formule générale (I), et, pour inclure des substances actives non solubles dans l'eau, on dissout la substance active en même temps que les lipides, et, pour inclure des substances actives solubles dans l'eau, on ajoute au film lipidique une solution tampon aqueuse qui contient la substance active soluble dans l'eau.

**Revendications pour l'Etat contractant suivant : GR**

**1.** Liposomes, caractérisés en ce qu'ils contiennent au moins 1 % en moles d'un composé présentant un excès de charge positive de formule générale (I)

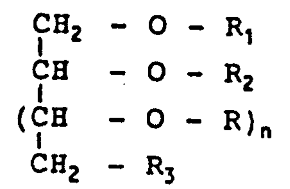

(I)

dans laquelle
$R_1$ représente un alcanoyle ou un alkyle ayant chacun 14 à 18 atomes de carbone, un oléoyle ou un oléyle,
$R_2$ représente le groupe PNN

PNN =

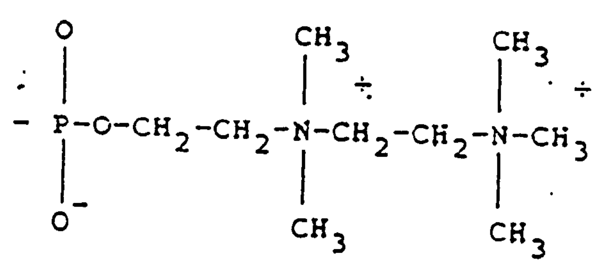

$R_3$ signifie -O-$R_1$, -O-PNN, -O-Gly, $NH_3^+$, $NH_2CH_3^+$, $NH(CH_3)_2^+$ ou $N(CH_3)_3^+$,
R possède l'une des significations données pour $R_1$, $R_2$ ou $R_3$, et
n représente un nombre entier de 0 à 3, sous réserve que la molécule contienne l'un des groupes à charge positive indiqués.

**2.** Liposomes selon la revendication 1, caractérisés en ce que, dans la formule (I), n est 0.

3. Liposomes selon la revendication 1 ou 2, caractérisés en ce qu'ils contiennent 1 à 30 % en moles d'un composé de formule (I).

4. Liposomes selon l'une des revendications 1 à 3, caractérisés en ce qu'ils contiennent 5 à 15 % en moles d'un composé de formule (I).

5. Procédé de préparation de liposomes selon l'une des revendications 1 à 4, caractérisé en ce que l'on transforme en une suspension de lipides au moins 1 % en moles d'un composé de formule générale (I) avec les autres constituants des liposomes en une quantité qui donne 100 % en moles avec le composé de formule générale (I), on soumet la suspension de lipides à un traitement thermique préalable et on transforme ensuite la suspension de lipides ainsi prétraitée à la chaleur en liposomes de façon connue en soi par pressage et centrifugation.

6. Procédé de préparation d'une composition de médicament, caractérisé en ce que l'on travaille suivant le procédé selon la revendication 5 pour préparer les liposomes selon l'invention qui contiennent au moins 1 % en moles d'un composé de formule générale (I), et, pour inclure des substances actives non solubles dans l'eau, on dissout la substance active en même temps que les lipides, et, pour inclure des substances actives solubles dans l'eau, on ajoute au film lipidique une solution tampon aqueuse qui contient la substance active soluble dans l'eau.

7. Procédé selon la revendication 6, caractérisé en ce que, dans la formule (I), n est 0.

8. Procédé selon la revendication 6 ou 7, caractérisé en ce que l'on utilise des liposomes qui contiennent 1 à 30 % en moles d'un composé de formule (I).

9. Procédé selon la revendication 8, caractérisé en ce que l'on utilise des liposomes qui contiennent 5 à 15 % en moles d'un composé de formule (I).

10. Utilisation d'un composé ayant la formule générale (I) donnée dans la revendication 1, dans laquelle $R_1$, $R_2$, $R_3$, R et n ont la signification donnée dans la revendication 1 ou 2, pour la préparation d'un médicament pour le traitement de maladies dans le domaine du foie.

**Revendications pour l'Etat contractant suivant : ES**

1. Procédé de préparation de liposomes, caractérisé en ce que l'on transforme en une suspension de lipides au moins 1% en moles d'un composé de formule générale (I)

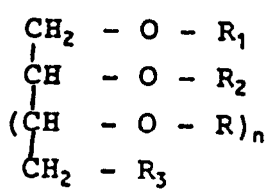

(I)

dans laquelle
$R_1$ représente un alcanoyle ou un alkyle ayant chacun 14 à 18 atomes de carbone, un oléoyle ou un oléyle,
$R_2$ représente le groupe PNN

PNN =

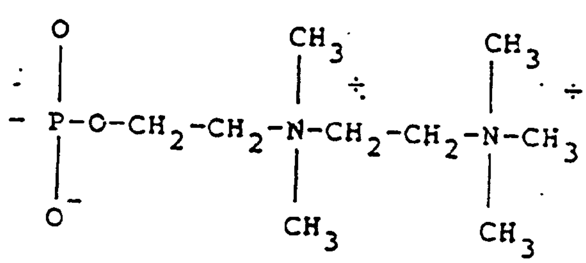

$R_3$ signifie -O-$R_1$, -O-PNN, -O-Gly, $NH_3^+$, $NH_2CH_3^+$, $NH(CH_3)_2^+$ ou $N(CH_3)_3^+$,
R possède l'une des significations données pour $R_1$, $R_2$ ou $R_3$, et
n représente un nombre entier de 0 à 3,

avec les autres constituants des liposomes en une quantité qui donne 100 % en moles avec le composé de formule générale (I), on soumet la suspension de lipides à un traitement thermique préalable et on transforme ensuite la suspension de lipides ainsi prétraitée à la chaleur en liposomes de façon connue en soi par pressage et centrifugation.

**2.** Procédé selon la revendication 1, caractérisé en ce que, dans la formule (I), n est 0.

**3.** Procédé selon la revendication 1 ou 2, caractérisé en ce que l'on introduit 1 à 30 % en moles d'un composé de formule (I).

**4.** Procédé selon l'une des revendications 1 à 3, caractérisé en ce que l'on introduit 5 à 15 % en moles d'un composé de formule (I).

**5.** Procédé de préparation d'une composition de médicament pour le traitement de maladies du foie, contenant une ou plusieurs substances actives qui doivent être transportées vers le foie et qui sont incluses dans des liposomes, caractérisé en ce que l'on travaille suivant le procédé selon la revendication 1 pour préparer les liposomes qui contiennent au moins 1 % en moles d'un composé de formule générale (I), et, pour inclure des substances actives non solubles dans l'eau, on dissout la substance active en même temps que les lipides, et, pour inclure des substances actives solubles dans l'eau, on ajoute au film lipidique une solution tampon aqueuse qui contient la substance active soluble dans l'eau.

**6.** Procédé selon la revendication 5, caractérisé en ce que, dans la formule (I), n est 0.

**7.** Procédé selon la revendication 5 ou 6, caractérisé en ce que les liposomes contiennent 1 à 30 % en moles d'un composé de formule (I).

**8.** Procédé selon l'une des revendications 5 à 7, caractérisé en ce que les liposomes contiennent 5 à 15 % en moles d'un composé de formule (I).